# EUROPEAN PATENT APPLICATION

(11) **EP 1 787 645 A1**
(43) Date of publication of application: **23.05.2007**
(21) Application number: 05292450.3
(22) Date of filing: 18.11.2005
(51) Int. Cl.: A61K 31/436, A61K 38/13, A61P 35/02

(54) **New method for treating cancer based on the modulation of the calcineurin and/or the calcineurin/NFAT pathway**

(71) Applicant: Institut Curie, 75248 Paris Cedex 05 (FR); CENTRE NATIONAL DE LA RECHERCHE SCIENTIFIQUE, 75794 Paris Cedex 16 (FR)
(72) Inventor: Ghysdael, Jacques, 91190 Gif-sur-Yvette (FR); Medyouf, Hind, 94800 Villejuif (FR); Guillemin, Marie-Claude, 78400 Chatou (FR)
(74) Representative: Gallois, Valérie

(57) **Abstract**

The present invention relates to methods for treating a haematopoietic tumor by administering a drug inhibiting the calcineurin and/or the calcineurin/NFAT pathway, alone or in combination with others cancer therapy, pharmaceutical compositions useful in such methods, and screening methods for identifying a compound useful for treating a haematopoietic tumor.

## Description

### FIELD OF THE INVENTION

The present invention relates to methods for treating a haematopoietic tumor, pharmaceutical compositions useful in such methods, and screening methods for identifying a compound useful for treating a haematopoietic tumor.

### BACKGROUND OF THE INVENTION

Acute lymphoblastic leukemia (ALL) is the most common malignancy in children < 10 years-old whereas its occurrence in adults steadily increases with age. Non Hogdkin lymphoma (NHL) is the most common hematopoietic malignancy and is currently the 5^{th} most common cancer in the western world. It includes a number of clinical entities, as defined in the REAL or WHO classification, with a significant clinical overlap between precursor T- and B-cell lymphoblastic lymphoma and ALL. Remission in these clinical entities is induced by intensive combination chemotherapy (e.g. CHOP in disseminated NHL). Relapse is rare in childhood ALL but frequent in adult ALL. In NHL, depending on the entity, only 40 to 70 % of patients achieve long term remission using CHOP or CHOP-based primary chemotherapy. Improvement of existing treatment regimens is therefore required. Approaches along these lines include the search for novel clinical, histological and molecular prognostic factors, the use of high dose chemotherapy followed by hematopoietic stem cell transplantation in relapsed cases, the search and integration of novel therapies into existing treatment strategies. In addition, the repeated multidrug treatment of ALL and NHL is associated with severe immediate toxicity and poor quality of life and long term sequelae, including other cancers. Lymphoma/leukemia patients would therefore benefit greatly from novel therapeutic approaches, in particular those that directly target the molecular mechanisms responsible for tumor cell survival and proliferation, or those involved in the essential interactions between tumor cells and their micro-environment.

Calcineurin (PP2B) is an ubiquitously expressed serine/threonine protein phosphatase that is involved in many biological processes and which is essential for life. Calcineurin is a heterodimer composed of a catalytic subunit (CnA ; three isoforms) and a regulatory subunit (CnB ; two isoforms). Besides its catalytic domain, CnA includes a CnB-binding helical domain, a calmodulin binding region and an auto-inhibitory domain (AID) (1). Engagement of cell surface receptors coupled to phospholipase C activation results in the generation of inositol(1,4,5)trisphosphate (InsP3) and diacylglycerol (DAG). While DAG is involved in PKC activation, InsP3 mediates the release of calcium from internal stores. In turn, store depletion induces the opening of specific store-operated channels that result in the influx of extracellular calcium. This increase in calcium ions concentration induces the binding of calmodulin to calcineurin, the release of calcineurin from AID inhibition and activation of its phosphatase activity. Mutation by homologous recombination of the ubiquitously expressed CnB1 gene results in the suppression of calcineurin activity in all somatic tissues and in embryonic lethality at day 11.5 of mouse development (2). Interestingly, the CnB1 mutant phenotype phenocopies that of a double knockout of NFAT3+4, indicating that NFAT proteins are major downstream substrates of calcineurin in mouse development (2).

The NFAT family of transcriptional regulators includes NFAT1, NFAT2, NFAT3, NFAT4 and NFAT5. Except for NFAT5, the other NFAT proteins are activated by cell surface receptors coupled to phospholipase C activation and to store-operated Ca²⁺ entry, typically the pre-TCR and the T cell antigen receptor in T lymphoid cells (for review, see (3)). NFAT1-4 share a similar modular structure, including N-terminal and C-terminal activation domains; a central Rel-homology domain that mediates DNA binding; a regulatory domain that includes multiple serine phosphorylation sites (4) and a calcineurin docking domain. The major docking site of calcineurin is localized in the N-terminal region of the regulatory domain and is centered over a critical PxIxIT motif. In resting cells, NFAT1-4 are fully phosphorylated in their regulatory domain, are cytosolic and in a conformation inhibiting their DNA binding activity. Ca²⁺/calmodulin-induced activation of calcineurin induces the concerted dephosphorylation of NFATs, their nuclear accumulation and the activation of their DNA binding activity. Several constitutive and signal-induced export protein kinases have been implicated in the maintenance of NFAT hyperphosphorylation in resting cells and in their nuclear re-phosphorylation after signal-evoked dephosphorylation, including casein kinase 1, glycogen synthase kinase 3, Jun kinase 1 (JNK1) and the related p38. NFAT1-4 bind DNA as monomer to their cognate A/TGGAA binding site, as dimers at NFκB-like response elements and as cooperative complexes (e.g. NFAT/AP1 ; NFAT/STAT4; NFAT/MAF/GATA3) on composite DNA response elements in specific cell lineages and/or in response to the activation of specific receptors.

NFAT1-4 play critical roles in many developmental processes and in the immune response. The best characterized function of the calcineurin/NFAT pathway is its essential role in T cell activation following co-engagement of the TCR and co-activator receptors like CD28 by antigen-presenting cells. In this response, NFAT1 and NFAT2 play a redundant role and activate the expression of a number of activation-specific genes through their binding, together with c-JUN/C-FOS to composite NFAT/AP1 response elements in the promoter region of these genes ((5) and references therein). Remarkably, NFAT1 plays a prominent role in the inhibition of TCR signaling in T cells subjected to an anergizing stimuli e.g. Ca²⁺ signaling without concomitant PKC/MAPkinase activation. In that situation, NFAT1 regulates the transcription of a different set of genes either through its ability to bind specific response elements as homodimer, or in synergy with transcriptional partners different from AP1. The calcineurin/NFAT pathway, most specifically NFAT4 plays a major role in T cell development, in particular in positive selection during the transition of immature CD4CD8 double positive (DP) thymocytes to mature CD4 and CD8 SP T cells (6) and in the functional differentiation of T cells, most notably in both Th1 and Th2 differentiation from naive T helper cells through cooperation with specific STATs and lineage-specific transcription factors (for review, see (7))

Since calcineurin and its downstream NFAT substrates have a central role in T cell activation, this pathway is a critical target for therapeutic control of pathological immune responses (for review, see (8)). Two inhibitors of calcineurin, cyclosporinA and FK506 (Prograf) act by binding to specific intracellular receptors, cyclophilin and FKBP12, respectively. The respective drug/receptor complexes binds calcineurin and inhibit its activity, resulting in the full rephosphorylation of NFATs and their accumulation in the cytoplasm. Both CsA and FK506 are extensively used as immunosuppressive agents in human medicine to facilitate allograft survival and autoimmune diseases. More specific inhibitors of NFAT activation have been generated, in particular a high affinity version of the PXIXIT domain, known as the VIVIT peptide; when expressed in cells as a GFP fusion, this peptide selectively blocks NFAT dephosphorylation and NFAT-dependent transcription (9). Recently, several pharmacological compounds have been identified that block the NFAT-calcineurin interaction, but are at present of limited interest in vivo due to cell toxicity (10).

Although critically important in many aspects of T cell survival, activation and proliferation, the calcineurin/NFAT pathway has so far not been involved in T cell lymphoma/leukemia development.

More generally, a role of this pathway in tumorigenesis is suspected but not clear and not proven. Indeed, in vitro studies have shown that (i) expression of a constitutively nuclear mutant of NFAT2 interferes with the differentiation of the 3T3-L1 fibroblastic cell line into adipocytes and induces morphological transformation of these cells and their growth as tumors in immunosuppressed mice (11); (ii) both NFAT1 and NFAT5 expression is induced in response to integrin signaling in a breast carcinoma-derived cell line and participate in the activation of cell migration and invasion of matrigel, but this response is not dependent on calcineurin activity (12); (iii) NFAT2 is nuclear in a subset of human leukemia, including diffuse large B-cell lymphoma (LBCL) and is involved in cell growth of LBCL cell lines in vitro (13).

The patent application US2005100897 describes that NFAT may be involved in promoting carcinoma invasion based on in vitro observations. NFAT1 and NFAT5 are expressed at high levels and are constitutively active in cell lines derived from human breast and colon carcinomas. They showed that an increase in matrigel invasion can be blocked in vitro with a dominant negative NFAT mutant, but not cyclosporin A or FK506.

### SUMMARY OF THE INVENTION

The inventors have found that the calcineurin and/or the calcineurin/NFAT pathway is activated in lymphoid malignancies. The activation of the calcineurin/NFAT pathway in these cancer cells was difficult to observe because the activation of NFAT disappears as soon as the cells are maintained in culture. More particularly, the activation of the calcineurin/NFAT pathway in these cancer cells is based on assessing the dephosphorylation of NFAT.

The inventors have shown the calcineurin and/or the calcineurin/NFAT pathway is a target of therapeutic interest in lymphoid malignancies. Surprisingly, inhibitors of calcineurin/NFAT pathway are shown to be of therapeutic interest to control the evolution of lymphoid malignancies, by affecting either the tumor cell itself and/or its stromal micro-environment.

Therefore, the present invention concerns the use of a drug inhibiting the calcineurin and/or the calcineurin/NFAT pathway for the preparation of a medicament for treating a haematopoietic tumor.

The present invention also concerns the use of a drug inhibiting the calcineurin and/or the calcineurin/NFAT pathway for the preparation of a medicament for increasing the efficiency of a treatment of a haematopoietic tumor.

In a preferred embodiment, the drug inhibiting the calcineurin and/or the calcineurin/NFAT pathway is a drug that inhibits NFAT dephosphorylation, such as drug that inhibits directly or indirectly calcineurin, a drug that inhibits the interaction between calcineurin and NFAT, a drug that activates a NFAT kinase and a combination thereof. Preferably, drug inhibiting the calcineurin and/or the calcineurin/NFAT pathway is a calcineurin inhibitor such as cyclosporin A and FK506.

In a preferred embodiment, the haematopoietic tumor is a lymphoma and/or a leukemia.

In a preferred embodiment, the drug inhibiting the calcineurin and/or the calcineurin/NFAT pathway is used in combination with a cancer therapy. Said cancer therapy can be selected from the group consisting of a cancer chemotherapy, an immunotherapy, a radiotherapy, a hormone or cytokine therapy, any other therapeutic method used for the treatment of a haematopoietic tumor and a combination thereof.

In a preferred embodiment, the subject to be treated presents dephosphorylated NFAT in cells of the haematopoietic tumor isolated from said subject.

The present invention further concerns a product containing a drug inhibiting the calcineurin and/or the calcineurin/NFAT pathway and an anticancer drug as a combined preparation for simultaneous, separate or sequential use in a cancer therapy.

The present invention also concerns a pharmaceutical composition comprising a drug inhibiting the calcineurin and/or the calcineurin/NFAT pathway and an anticancer drug.

In a preferred embodiment, said drug inhibiting the calcineurin and/or the calcineurin/NFAT pathway is a drug that inhibits NFAT dephosphorylation, such as drug that inhibits calcineurin, a drug that inhibits the interaction between calcineurin and NFAT, a drug that activates a NFAT kinase and a combination thereof. Preferably, the drug inhibiting the calcineurin and/or the calcineurin/NFAT pathway is the calcineurin inhibitor such as cyclosporin A and FK506.

The present invention further concerns a method for staging or characterizing a haematopoietic tumor in a subject comprising determining the phosphorylation state of NFAT in cells of the haematopoietic tumor isolated from said subject. A dephosphorylated NFAT is an activated NFAT involved in cancer development whereas a phosphorylated NFAT is an inactivated NFAT. In a particular embodiment, a dephosphorylated NFAT is related to an invasive capacity, a metastastic potential, and/or a relapse probability.

In addition, the present invention concerns a method for screening, identifying or selecting a drug for treating a haematopoietic tumor, comprising contacting in vitro or in vivo a test compound with a NFAT polypeptide under conditions in which calcineurin is able to dephosphorylate said NFAT polypeptide and determining whether said test compound affects the phosphorylation state of NFAT.

### LEGEND TO THE FIGURES

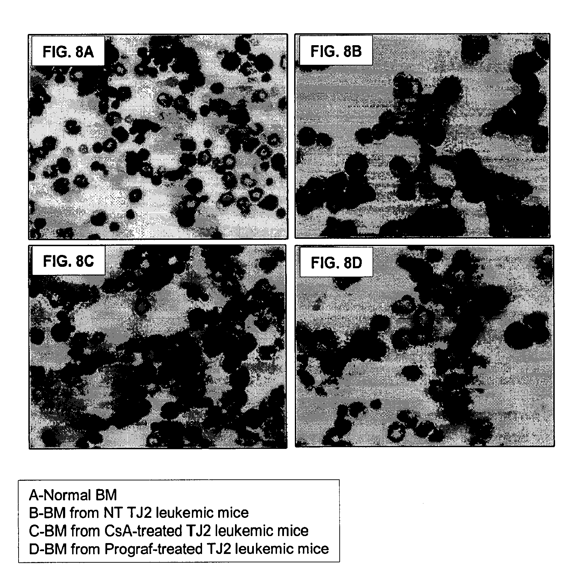

Figure 1. NFAT1 expression and activation in TEL-JAK2 leukemia.
**Fig. 1A:** Whole cell extracts of control thymocytes (WT) and Tg TEL-JAK2 leukemic cells (TJ2) (14) isolated from an invaded thymus were analyzed by SDS/PAGE and western blot using a NFAT1-specific antibody (upper panel). Samples were normalized using an either an anti-STAT5 (middle) or an anti-ERK2 antibody lower panel). Fig. 1B : same as in Fig. 1A, except that cells used in lanes 3 and 4 were maintained in tissue culture in the presence of cyclosporin A (CsA), or ionomycin (Iono), as indicated.
Figure 2. TgTEL-JAK2 leukemic cells express activated NFATs : analysis by electrophoretic mobility shift assays (EMSA)

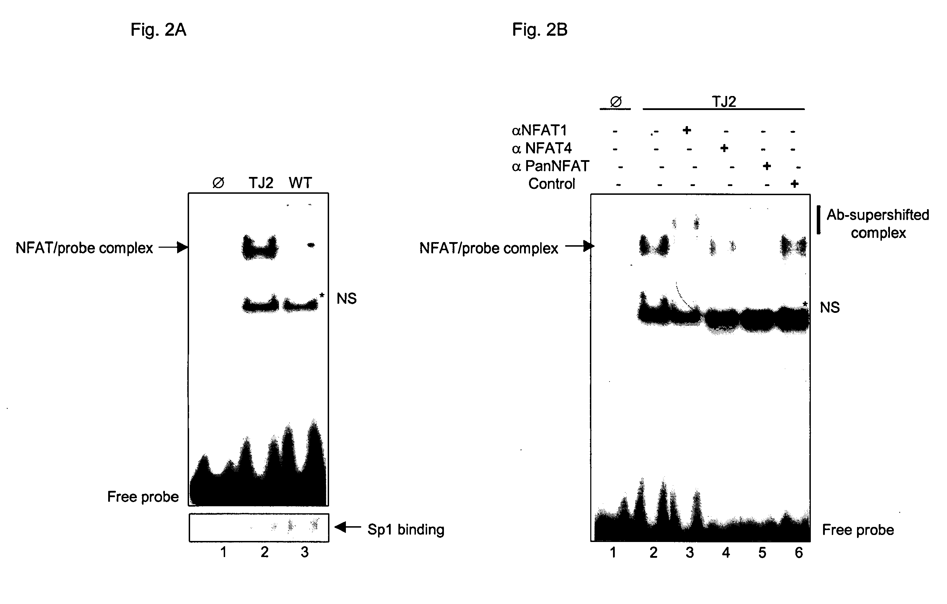

**Fig. 2A :** Nuclear extracts obtained from control thymocytes (WT) and TgTEL-JAK2 leukemic cells were analyzed for NFAT DNA binding activity by EMSA, using as DNA probe a double stranded oligonucleotide corresponding to the mouse IL2 promoter -45 NFAT-response element (top panel). Migration of the probe in the absence of any extract is shown in lane 1. The bottom panel displays the binding activity of the nuclear extracts used to a probe specific of the ubiquitously-expressed Sp1. Note that equal binding to the Sp1 probe is observed in the WT and TgTEL-JAK2 nuclear extracts. **Fig. 2B:** as in Fig. 2A, except that the DNA binding reaction mixture included 1 µl of the indicated NFAT antibiodies (anti-NFAT1 ; anti-NFAT4) or a pan-NFAT antibody, specific of NFAT1-4. The negative control used is a c-Rel-specific antibody.
Figure 3. Activated Notch-induced T cell leukemia activate NFAT

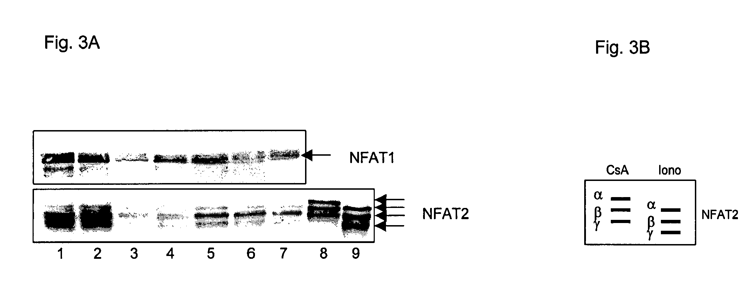

**Fig. 3A :** tumor cells from a series of independent ICN1-induced T cell leukemia obtained directly from diseased mice (lanes 1-7), or maintained in culture for 60 minutes in either the presence of CsA (lane 8) or ionomycin (lane 9) were analyzed by western blot for expression and activation of NFAT1 (top panel) and NFAT2 (bottom panel), using antibodies specific for NFAT1 or NFAT2, respectively. Phosphorylated and de-phosphorylated isoforms are indicated by coloured arrows. **Fig. 3B :** Schematic representation of the different NFAT2 splicing isoforms and their relative migration in their fully phospshorylated (ionomycin) or dephosphorylated states (CsA).
Figure 4. NFAT activation is not under the sole control of TEL-JAK2 oncoprotein in TgTEL-JAK2 leukemic cells but requires a proper tumor micro-environment.

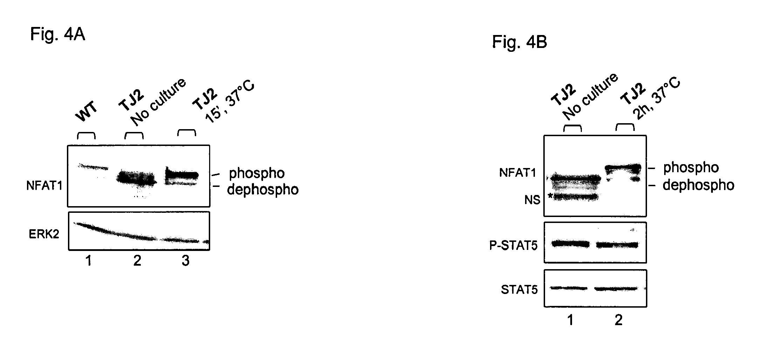

**Fig. 4A :** Western blot analysis of NFAT1 activation in total extracts from thymocytes (lane1) and TgTEL-JAK2 leukemic cells (lanes 2 and 3). Analysis of extracts prepared from leukemic cells directly obtained from diseased animals (lane 2) shows that, in contrast to normal thymocytes, NFAT1 is mainly present in a dephosphorylated, active state in TgTEL-JAK2 animals (compare lanes 1 and 2). However, after 15min in culture as isolated cells (lane 3), TgTEL-JAK2 leukemic cells exhibit rephosphorylation of the almost complete intracellular pool of NFAT1 proteins (compare lanes 2 and 3). **Fig. 4B :** Top : western blot analysis of NFAT1 activation in total extracts from TgTEL-JAK2 leukemic cells directly obtained from diseased animals (lane 1), or after 2 hours in culture (lane 2). Middle : western blot analysis of STAT5 activation TgTEL-JAK2 leukemic cells directly obtained from diseased animals (lanel), or after 2 hours in culture (lane 2), as analyzed using a STAT5 phosphotyrosine antibody. Bottom : analysis of STAT5 expression, using a STAT5-specific antibody. Note that under these conditions, TEL-JAK2 tyrosine kinase activity is maintained as shown by the phosphorylation of STAT5 (bottom panel).
Figure 5. Proper tumor micro-environment is required for NFAT activation in ICN1-induced leukemia and EBV-induced human lymphoma.

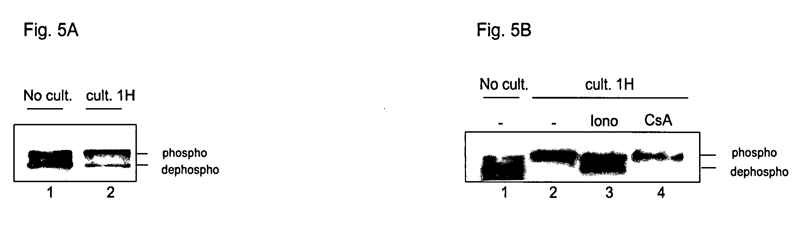

**Fig. 5A:** Western blot analysis of NFAT1 expression and activation in total extracts directly prepared from the leukemic cells obtained from ICN1-induced leukemia (lane 1), or the same cells maintained in culture for 1 hour (lane 2). Note that NFAT1 is in its phosphorylated (activated) form in ICN-1 leukemic cells and that activation is rapidly lost when leukemic cells are removed from their normal micro-environment and maintained in culture as isolated cells. **Fig. 5B :** Western blot analysis of NFAT1 expression and activation in total extracts directly prepared from the leukemic cells obtained from an EBV-induced human B cell lymphoma (lane 1), or the same cells maintained in culture as isolated cells for 1 hour without further treatment (lane 2), or in the presence of ionomcin (lane 3) or CsA (lane 4). Note that NFAT1 is activated in leukemic cells in situ, but that activation is lost when cells are removed from their normal tumoral micro-environment.
Figure 6. Calcineurin inhibitors cyclosporinA (CsA) and FK506 (Prograf) inhibit progression of TgTEL-JAK2 leukemia.

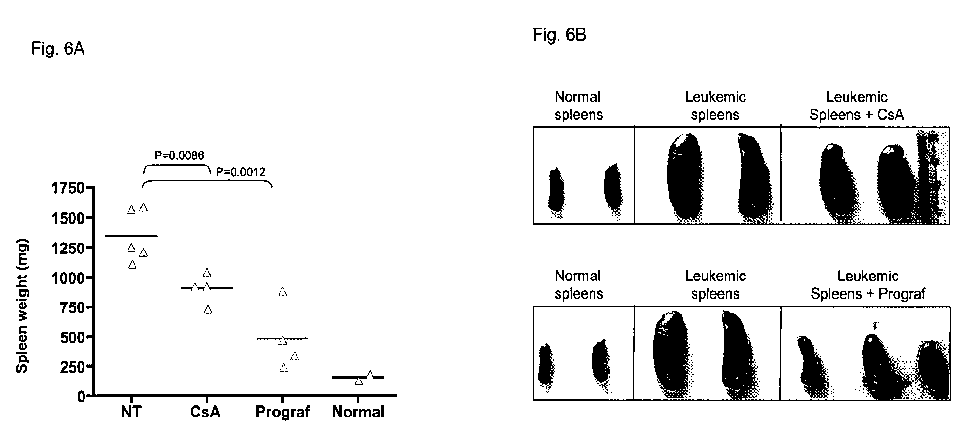

**Fig. 6A :** TgTEL-JAK2 leukemic cells were grafted into syngenic recipient mice. Under these conditions, leukemic cells engraft and proliferate in peripheral lymphoid organs and metastasize to non hematopoietic organs such as liver. (A) Three cohorts of mice were compared. Control (untreated, NT) ; mice treated with CsA ; mice treated with Prograf. Note that spleen invasion is inhibited by CsA and Prograf treatment (left), as analyzed by measuring spleen weight. The weight of age-matched control mice is shown for comparison (Normal). **Fig. 6B :** Pictures of representative spleens, as indicated in the legend. Normal spleen; Leukemic spleen (untreated); Leukemic spleen from CsA- and Prograf-treated mice.
Figure 7. CsA and Prograf treatment inhibits NFAT factors activation.
**Fig. 7A :** Western blot analysis of NFAT1 (top) and NFAT4 (middle) expression and phosphorylation in non treated (NT) and CsA-treated TgTEL-JAK2 leukemia. **Fig. 7B :** Western blot analysis of NFAT1 (top) and NFAT4 (middle) expression and phosphorylation in non treated (NT) and Prograf-treated TgTEL-JAK2 leukemia. Note the fast migrating (activated) forms of NFAT1 and NFAT4 in the untreated leukemia and the fully phosphorylated, inactive species in CsA- and Prograf-treated leukemias. Western blot anlysis of ERK expression (bottom) is shown as loading control.
Figure 8. CsA and Prograf treatment strongly interferes with leukemia progression.
   Imprints of: normal bone marrow (Fig. 8A), leukemic bone marrow from untreated TgTEL-JAK2 leukemic mouse (Fig. 8B) and bone marrow from CsA-treated (Fig. 8C) and Prograf-treated TgTEL-JAK2 leukemic mice (Fig. 8D) were stained with May-Grunwald-Giemsa. Note that the majority of cells in normal bone marrow (Fig. 8A) are of myeloid origin (granulocytic morphology); in contrast, the leukemic bone marrow obtained from an untreated animal is composed of an homogeneous population of T lymphoblastic cells that replaces the normal cells (Fig. 8B). Treatment with CsA (Fig. 8C) and Prograf (Fig. 8D) results in severe diminution in leukemic cells numbers and in the recovery of a cell composition and morphology close from that of normal bone marrow.

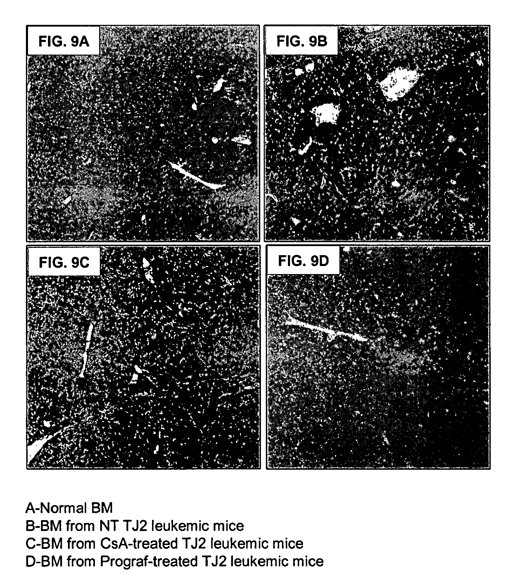

Figure 9. CsA and Prograf treatment inhibits invasion of leukemic cells in non-hematopoietic organs.
   HES (Hematoxylin Eosin Safran) staining of parafin- included sections of liver from either normal mouse (Fig. 9A), a leukemic, untreated mouse (Fig. 9B), a leukemic CsA-treated mouse (Fig. 9C) and a leukemic, Prograf-treated mouse (Fig. 9D). Fig. 9A shows the normal structure of the liver parenchyma. The untreated TgTEL-JAK2 leukemic mouse shows massive infiltration of leukemic cells (stained in blue) in the liver parenchyma through the portal areas and sinusoids (Fig. 9B). Tretment with CsA-(Fig. 9C) or Prograf (Fig. 9D) shows severe reduction of liver invasion by leukemic cells.

### DETAILED DESCRIPTION OF THE INVENTION

The Inventors have characterized a fusion between TEL and the 3' part of the gene encoding the JAK2 protein kinase in a case of childhood T cell ALL carrying a t(9;12) chromosomal translocation. The resulting chimeric gene encodes a TEL-JAK2 fusion protein in which the 336 amino-terminal residues of TEL are fused to the catalytic domain of JAK2, resulting in the constitutive activation of TEL-JAK2 tyrosine kinase activity. TEL-JAK2 is a strong oncogene in vivo since its targeted expression in the lymphoid lineage of transgenic mice results in a highly invasive lymphoma/leukemia (14).

The inventors have now found that the calcineurin/NFAT pathway is activated in TgTEL-JAK2 leukemic cells. Further analyses have shown that (i) the calcineurin/NFAT pathway is activated in a number of mouse models of lymphoma/leukemia induced by other human oncogenic proteins including activated Notch, overexpressed Myc and in a xenograft model of Hodgkin-like B cell lymphoma; (ii) that activation of the calcineurin/NFAT pathway is observed when tumour cells are maintained in vivo but is generally lost in vitro, suggesting that it is not under direct control of the primary oncogene activated in these haematopoietic malignancies; (iii) using the well-characterized model of TEL-JAK2-induced T cell leukemia/lymphoma, that in vivo inhibition of calcineurin by treatment of mice with CsA or FK506 result in the complete inactivation of NFAT and inhibition of tumor cell expansion and invasion.

The present data show that the calcineurin and/or the calcineurin/NFAT pathway is a target of therapeutic interest in lymphoid malignancies. They furthermore show that two inhibitors of calcineurin widely used in other indications in human medicine, namely CsA and FK506, could be of therapeutic interest to control the evolution of leukemia and lymphoma, by affecting either the tumor cell itself and/or its stromal micro-environment. In particular, the inventors showed that the cancer cells display a persistent dephosphorylation of NFAT.

### Definition

Where "comprising" is used, this can preferably be replaced by "consisting essentially of", more preferably by "consisting of".

Whenever within this whole specification "treatment of a haematopoietic tumor" or the like is mentioned with reference to a drug inhibiting the calcineurin and/or the calcineurin/NFAT pathway, there is meant:
a) a method of treatment (=for treating) of a haematopoietic tumor, said method comprising the step of administering (for at least one treatment) a drug inhibiting the calcineurin and/or the calcineurin/NFAT pathway, (preferably in a pharmaceutically acceptable carrier material) to a subject, especially a human, in need of such treatment, in a dose that allows for the treatment of said haematopoietic tumor (=a therapeutically effective amount);
b) the use of a drug inhibiting the calcineurin and/or the calcineurin/NFAT pathway for the treatment of a haematopoietic tumor; or a drug inhibiting the calcineurin and/or the calcineurin/NFAT pathway, for use in said treatment (especially in a human);
c) the use of a drug inhibiting the calcineurin and/or the calcineurin/NFAT pathway for the manufacture of a pharmaceutical preparation for the treatment of a haematopoietic tumor;
d) a pharmaceutical preparation comprising a dose of a drug inhibiting the calcineurin and/or the calcineurin/NFAT pathway that is appropriate for the treatment of a haematopoietic tumor; and/or
e) a product containing a drug inhibiting the calcineurin and/or the calcineurin/NFAT pathway and an anticancer drug as a combined preparation for simultaneous, separate or sequential use in the treatment of a hematopoietic tumor.

The present invention concerns the use of a drug inhibiting the calcineurin and/or the calcineurin/NFAT pathway for the preparation of a medicament for treating a hematopoietic tumor. In a particular embodiment, the subject to be treated presents dephosphorylated NFAT in cells of the haematopoietic tumor isolated from said subject.

The present invention further concerns a method for treating a hematopoietic tumor in a subject comprising administering a therapeutic amount of a drug inhibiting the calcineurin/NFAT pathway. Optionally, the method for treating a hematopoietic tumor in a subject comprises a previous step of determining the phosphorylation state of NFAT in cells of the haematopoietic tumor isolated from said subject. Indeed, presence of a dephosphorylated NFAT is indicative of an efficiency of the drug inhibiting the calcineurin and/or the calcineurin/NFAT pathway for treating said haematopoietic tumor.

The inventors have shown that a drug which inhibits the calcineurin and/or the calcineurin/NFAT pathway induces apoptosis of cancer cells and inhibits the proliferation of cancer cells. Therefore, this drug is of a great interest to block the progression of the cancer, in particular the spreading and the growth of cancer. This drug can also provides a cancer regression.

The present invention also concerns the use of a drug inhibiting the calcineurin and/or the calcineurin/NFAT pathway for the preparation of a medicament for increasing the efficiency of a treatment of a hematopoietic tumor. Preferably, the treatment of a hematopoietic tumor can be a cancer chemotherapy, an immunotherapy, a radiotherapy, a hormone or cytokine therapy, any other therapeutic method used for the treatment of a haematopoietic tumor or a combination thereof. More preferably, the treatment of a hematopoietic tumor is a cancer chemotherapy.

Preferably, said subject is a mammal. More preferably, said subject is a human.

NFAT (Nuclear Factor of Activated T-cells or NFATC) can be selected from the group consisting of NFAT1 (also called NFATP and NFATC2, Unigene Hs.356321), NFAT2 (also called NFATC 1 and NFATC, Unigene Hs.534074), NFAT3 (also called NFATC4, Unigene Hs.77810), NFAT4 (also called NFATC3 and NFATX, Unigene Hs.341716) and any combination thereof. In a preferred embodiment, said NFAT is selected from the group consisting of NFAT1, NFAT2, and NFAT4. The disclosures of the Unigene files corresponding to the aforementioned accession numbers are incorporated herein by reference.

More specifically, the present invention can be utilized for the treatment of a hematopoietic tumor. The hematopoietic tumor can be a hematopoietic tumor of lymphoid lineage, including leukemia, acute lymphocytic leukemia, acute lymphoblastic leukemia, B-cell lymphoma, T-cell lymphoma, Hodgkins lymphoma, non-Hodgkins lymphoma, hairy cell lymphoma and Burkitt lymphoma or a hematopoietic tumor of myeloid lineage, including acute and chronic myelogenous leukemias and promyelocytic leukaemia. The cancer can be a primary tumor or a metastasis. The cancer to treat can also be a relapse.

According to the present invention, a drug inhibiting the calcineurin and/or the calcineurin/NFAT pathway leads to an inactivation of NFAT, e.g. a phosphorylation of NFAT. Indeed, the inventors have observed an activation of NFAT in the primary cancer cells isolated from a human subject. NFAT activation includes protein-protein interaction between calcineurin and NFAT, dephosphorylation of NFAT by calcineurin, and translocation of NFAT to the nucleus. Therefore, several kinds of drugs inhibiting the calcineurin and/or the calcineurin/NFAT pathway can be contemplated by the present invention. The first one is a drug inhibiting calcineurin. A second kind of drugs can be a compound which inhibits the interaction between NFAT and calcineurin. A third kind of drugs can be a drug increasing the phosphorylation of NFAT. This list is not intended to be limitative.

As the calcineurin is involved in the dephosphorylation of NFAT proteins, the drug inhibiting the calcineurin and/or the calcineurin/NFAT pathway can be an inhibitor of calcineurin. Calcineurin inhibitors are already used in therapy as an immunosupressant to prevent rejection following organ transplantation. In immunosuppressive therapy, the calcineurin inhibitors are used in high doses for long term treatment. Such drugs include, but are not limited thereto cyclosporin A (Novartis International AG, Switzerland), FK506 (Fujisawa Healthcare, Inc., Deerfield, IL, USA), FK520 (Merck & Co, Rathway, NJ, USA), L685,818 (Merck & Co), FK523, and 15-0-DeMe-FK-520 (Liu, Biochemistry, 31:3896-3902 (1992)). W02005087798 describes cyclosporine derivative inhibiting calcineurin.

Calcineurin is a serine/threonine protein phosphatase, which is a heterodimer composed of a catalytic subunit (Calcineurin A) and a regulator subunit (Calcineurin B). Then, the activity of calcineurin can also be inhibited by blocking its expression, in particular the expression of one of its subunit. In a preferred embodiment, the activity of calcineurin can also be inhibited by blocking the expression of the regulator subunit B. The expression can be blocked by any mean known by one skilled in the art, e.g., by chemically synthesized oligonucleotides such as antisense oligonucleotides, ribozymes, short interfering RNA (siRNA) and short hairpin RNA (shRNA). Antisense oligonucleotides are short single-strand molecules that are complementary to the target mRNA and typically have 10-50 mers in length, preferably 15-30 mers in length, more preferably 18-20 mers in length. Antisense oligonucleotides are preferably designed to target the initiator codons, the transcriptional start site of the targeted gene or the intron-exon junctions (for review, 16). Ribozymes are single stranded RNA molecules retaining catalytic activities. The mechanism of ribozyme action involves sequence specific interaction of the ribozyme molecule to complementary target RNA, followed by an endonucleolytic cleavage. The ribozyme is engineered to interact with the target RNA of interest comprising a cleavage NUH triplet, preferentially GUC (for review, 17). siRNA are usually 21 or 23 nucleotides long, with a 19 or 21 nucleotides duplex sequence and 2 nucleotides-long 3' overhangs. shRNA are designed with the same rules than for a sequence encoding a siRNA excepting several additional nucleotides forming a loop between the two strands of the siRNA. (For review 18)

Alternatively, the activity of calcineurin can be inhibited by a compound that inhibits the interaction between the calcineurin subunits, in particular the interaction between subunits A and B. The activity of calcineurin can be inhibited by a compound that inhibits the interaction between the calcineurin and calmodulin. Calcineurin inhibition can also be obtained by activation of endogenous inhibitors of calcineurin, including cabin1, calcipressins and AKAP79.

The drug inhibiting the calcineurin and/or the calcineurin/NFAT pathway can be a compound that inhibits the interaction between calcineurin and NFAT. Such a compound has been described in the US patent 6,686,450 which describes a polypeptide called Cabin 1 and fragment thereof that inhibit the interaction between calcineurin and NFAT, thereby inhibing the dephopshorylation of NFAT by calcineurin. The patent application WO2004/069200 disclosed peptides derived from NFAT capable of specifically inhibiting the interaction between calcineurin and NFAT, thereby inhibing the dephopshorylation of NFAT by calcineurin. One example of such a peptide is a peptide comprising or consisting to the amino acid sequence MAGPHPVIVITGPHEE. This patent application also describes small compounds capable of inhibiting the dephosphorylation of NFAT by calcineurin, for example INCA-1, INCA-2 and INCA-6.

The drug inhibiting the calcineurin/NFAT pathway can be a compound that stimulates a kinase which phosphorylates NFAT. Preferably, said NFAT kinase is glycogen synthase kinase-3 (GSK-3), casein kinase I, protein kinase A (PKA) or the MAPKinases JNK and p38. Therefore, the drug can be an agent that stimulates any one of these kinases. The activity of a kinase can be modulated by modulating the protein level of the kinase. For example, increasing the activity of a kinase can be accomplished by increasing the endogenous protein level of the kinase, such as by increasing transcription of the kinase. Alternatively, the activity of a kinase can be increased by introducing a kinase into a cell, such as by introducing a nucleic acid encoding the kinase. Furthermore, a NFAT kinase can also be activated by an intracellular agonist. For instance, cAMP is able to activate casein kinase 1.

Other drugs inhibiting the calcineurin and/or the calcineurin/NFAT pathway can be identified by screening methods already disclosed in the art. As illustration, the patent application WO2004/069200 disclosed screening methods useful for identifying drugs inhibiting the calcineurin/NFAT pathway. The US patent 6,171,818 describes screening methods useful for identifying drugs activating the phosphorylation of NFAT. The US patent 6,875,581 describes screening methods useful for identifying modulators of calcineurin activity.

In a preferred embodiment, the drug inhibiting the calcineurin and/or the calcineurin/NFAT pathway is a drug that inhibits NFAT dephosphorylation. Preferably, said drug is selected from the group consisting of a drug that inhibits calcineurin, a drug that inhibits the interaction between calcineurin and NFAT, a drug that activates NFAT kinase and a combination thereof.

The drug inhibiting the calcineurin and/or the calcineurin/NFAT pathway may be of various origin, nature and composition. It may be any organic or inorganic substance, such as a lipid, peptide, polypeptide, nucleic acid, small molecule, etc., in isolated or in mixture with other substances. In a preferred embodiment, the drug is a small molecule. In an other preferred embodiment, the drug is a peptide or a polypeptide. In an additional embodiment, the drug is a nucleic acid, e.g., an antisense, a siRNA, a ribozyme.

The drug inhibiting the calcineurin and/or the calcineurin/NFAT pathway can be used in association with a targeting moiety, the targeting moiety allowing to preferentially reach cancer cells rather than normal cell. Preferably, the targeting moiety allows the selective treatment of cancer cells. For example, B-subunit of Shiga toxin can be used as a cancer cell vectorization means (for more details, see WO2004016148).

According to the present invention, the drug inhibiting the calcineurin and/or the calcineurin/NFAT pathway can be used alone or in combination with usual cancer therapy. The cancer therapy can be selected from the group consisting of a cancer chemotherapy, an immunotherapy, a radiotherapy, a hormone or cytokine therapy, any other therapeutic method used for the treatment of a haematopoietic tumor and a combination thereof. Preferably, the cancer therapy is a cancer chemotherapy. In a preferred embodiment, the drug inhibiting the calcineurin and/or the calcineurin/NFAT pathway is used in combination with a cancer chemotherapy. The drug inhibiting the calcineurin and/or the calcineurin/NFAT pathway can be administered before, at the same time or after the cancer therapy. In a first embodiment, the drug inhibiting the calcineurin and/or the calcineurin/NFAT pathway and the anticancer drug can be administered by the same route. In an alternative embodiment, they are administered by different routes of administration.

The present invention concerns a method of treating a hematopoietic tumor in a subject comprising administering a therapeutic amount of a drug inhibiting the calcineurin and/or the calcineurin/NFAT pathway and a therapeutic amount of an anticancer drug. Optionally, the method for treating a hematopoietic tumor in a subject comprises a previous step of determining the phosphorylation state of NFAT in cells of the haematopoietic tumor isolated from said subject. Indeed, presence of a dephosphorylated NFAT is indicative of an efficiency of the drug inhibiting the calcineurin and/or the calcineurin/NFAT pathway for treating said haematopoietic tumor.

The present invention concerns a product containing a drug inhibiting the calcineurin and/or the calcineurin/NFAT pathway and an anticancer drug as a combined preparation for simultaneous, separate or sequential use in the treatment of a hematopoietic tumor.

The present invention concerns a pharmaceutical composition comprising a drug inhibiting the calcineurin and/or the calcineurin/NFAT pathway according to the present invention and an anticancer drug. Preferably, the drug inhibiting the calcineurin and/or the calcineurin/NFAT pathway is a drug that inhibits NFAT dephosphorylation, such as drug that inhibits calcineurin, a drug that inhibits the interaction between calcineurin and NFAT, and a drug that activates NFAT kinase. More preferably, the drug inhibiting the calcineurin and/or the calcineurin/NFAT pathway is a calcineurin inhibitor such as cyclosporin A or FK506. Such a pharmaceutical composition generally comprises a pharmaceutically acceptable carrier. By a pharmaceutically acceptable carrier is intended a carrier that is physiologically acceptable to the treated mammal while retaining the therapeutic properties of the drug with which it is administered. For example, a pharmaceutically acceptable carrier can be physiological saline solution. Other pharmaceutically acceptable carriers are known to one skilled in the art and described for instance in Remington: The Science and Practice of Pharmacy (20th ed., ed. A.R. Gennaro AR., 2000, Lippincott Williams & Wilkins).

Anticancer drugs interfere with cancer cells' ability to grow (multiply) or to survive. There are several types of drugs; each type interferes with the cell's ability to grow or survive in a different way. A brief description of several examples of drug types that are used to treat people with cancer follows. These chemotherapies are well-known by one skilled in the art.

A first class of drugs is DNA-damaging drugs which react with DNA to alter it chemically and prevent it from permitting cell growth. For instance, this kind of drug can be selected from the following group, but are not limited thereto : Busulfan (Myleran) ; Carboplatin (Paraplatin) ; Carmustine (BCNU) ; Chlorambucil (Leukeran) ; Cisplatin (Platinol) ; Cyclophosphamide (Cytoxan, Neosar) ; Dacarbazine (DTIC-Dome) ; Ifosfamide (Ifex) ; Lomustine (CCNU) ; Mechlorethamine (nitrogen mustard, Mustargen) ; Melphalan (Alkeran) ; and Procarbazine (Matulane).

A second class of drugs is antitumor antibiotics which interact directly with DNA in the nucleus of cells, interfering with cell survival. For instance, this kind of drug can be selected from the following group, but are not limited thereto : Bleomycin (Blenoxane) ; Daunorubicin (Cerubidine) ; Doxorubicin (Adriamycin, Rubex) ; Idarubicin (Idamycin) ; and Mitoxantrone (Novantrone).

A third class of drugs is antimetabolites which are chemicals that are very similar to the building blocks of DNA or RNA. They are changed from the natural chemical sufficiently so that when they substitute for it and block the cells' ability to form RNA or DNA, preventing cell growth. For instance, this kind of drug can be selected from the following group, but are not limited thereto : 5-azacytidine (AZA-CR) ; Cladribine (Leustatin) ; Cytarabine (cytosine arabinoside, Ara-C, Cytosar-U) ; Fludarabine (Fludara) ; Hydroxyurea (Hydrea) ; 6-mercaptopurine (Purinethol) ; Methotrexate (Rheumatrex) ; and 6-thioguanine (Thioguanine).

A fourth class of drugs is DNA-repair enzyme inhibitors which act on enzymes in the cell nucleus that normally repair injury to DNA. These drugs prevent the enzymes from working and make the DNA more susceptible to injury. For example, such drugs can be Etoposide (VP-16, VePesid) ; Teniposide (VM-26, Vumon) ; and Topotecan (Hycamptin).

A fifth class of drugs is drugs that prevent cells from dividing by blocking mitosis. For example, such drugs can be Vinblastine (Velban); Vincristine (Oncovin); and Paclitaxel (Taxol).

A sixth class of drugs is hormones that can kill lymphocytes. In high doses, these synthetic hormones, relatives of the natural hormone cortisol, can kill malignant lymphocytes. For example, such drugs can be Dexamethasone (Decadron); Methylprednisolone (Medrol) ; and Prednisone (Deltasone).

A seventh class of drugs is cell-maturing agents that act on a type of leukemia to induce maturation of leukemic cells. All-trans retinoic acid (ATRA) and Arsenic trioxide (Trisenox) can be cited as illustration.

An eighth class of drugs is biomodifiers based on natural products with exact mechanisms of action that are unclear, such as Interferon-alpha (Roferon A, Intron A).

A ninth class of drugs is monoclonal antibodies that target and destroy cancer cells with fewer side effects than conventional chemotherapy. Rituximab (Rituxan) and Gemtuzumab ozogamicin (Mylotarg) can be cited as illustration.

A tenth class of drugs is drugs with specific molecular targets. These agents are designed to block the specific mutant protein that initiates the malignant cell transformation, such as Imatinib mesylate (Gleevec, Glivec).

Of course, this list does not include every drugs being used or studied in clinical trials. Combinations of these drugs and drug groups often form the basis of treatment. Certain of these drugs have been found to be more or less active in a particular subtype of cancer, in particular leukemia, lymphoma or myeloma.

The compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. For example, the drug inhibiting the calcineurin and/or the calcineurin/NFAT pathway such as a calcineurin inhibitor is administered orally and the anticancer drug is administered intravenously. Alternatively, the drug inhibiting the calcineurin and/or the calcineurin/NFAT pathway such as a calcineurin inhibitor and the anticancer drug are both administered intravenously.

By a therapeutic amount is intended an amount of drug, alone or in combination with an anticancer drug, that is sufficient to inhibit cancer growth, progression or metastasis in vivo. The effective amount of a drug for the treatment of cancer varies depending upon the administration mode, the age, body weight, sex and general health of the subject. It is an amount that is sufficient to effectively reduce cell proliferation, tumor size, cancer progression or metastasis. It will be appreciated that there will be many ways known in the art to determine the therapeutic amount for a given application. For instance, the dose of FK506 can be from 0.001 mg/kg/day to 10 mg/kg/day, preferably between 0.01 and 10 mg/kg/day by oral administration and between 0.001 and 1 mg/kg/day by intravenous injection. The dose of cyclosporin A as oral formulation (Neoral; Sandimmune) can be from 0.1 mg/kg/day to 10 mg/kg/day.

In a particular embodiment, the composition comprising the drug inhibiting the calcineurin and/or the calcineurin/NFAT pathway is administered for a short period of time. In a particular embodiment, the period can be the period of the chemotherapy. Optionnally, the period can be from one day to one month. Optionally, the period of treatment can be repeated. The drug inhibiting the calcineurin and/or the calcineurin/NFAT pathway can be administered once a day, twice a day or more. In a preferred embodiment, the drug inhibiting the calcineurin and/or the calcineurin/NFAT pathway is administered so as to avoid an immunosuppresive effect. For example, this immunosuppresive effect can be obtained by adapting the dose (e.g. lower dose) or the period of treatment (e.g. shorter period).

The administration protocols for the cancer chemotherapy are well-known by one skilled in the art.

The present invention further concerns a method for staging or characterizing a hematopoietic tumor in a subject comprising determining the phosphorylation state of NFAT in cancer sample isolated from said subject. A dephosphorylated NFAT is an activated NFAT involved in cancer development whereas a phosphorylated NFAT is an inactivated NFAT. In a particular embodiment, a dephosphorylated NFAT is related to an invasive capacity, a metastastic potential, a relapse probability. The cancer sample from the patient is a body fluid, preferably a blood sample. In a preferred embodiment, the phosphorylation state of NFAT is assayed directly on the removed sample, without any culture step. Alternatively, the phosphorylation state of NFAT is on a short culture of the sample, preferably less than one hour.

The present invention also concerns a method of assessing the response of a subject to a treatment of a haematopoietic tumor with a drug inhibiting the calcineurin/NFAT pathway, comprising determining the phosphorylation state of NFAT in cancer sample isolated from said subject, a presence of a dephosphorylated NFAT being indicative of an efficiency of the drug inhibiting the calcineurin and/or the calcineurin/NFAT pathway for treating said haematopoietic tumor.

The present invention concerns a method for screening, identifying or selecting a drug for treating a haematopoietic tumor, comprising contacting in vitro or in vivo a test compound with a NFAT polypeptide under conditions in which calcineurin is able to dephosphorylate said NFAT polypeptide and determining whether said test compound affects the phosphorylation state of NFAT. In a particular embodiment, a NFAT polypeptide under conditions in which calcineurin is able to dephosphorylate said NFAT polypeptide is comprised into isolated cells or into cells of a test non-human animal.

The phosphorylation state can be assayed by different methods known by one skilled in the art. For example, as shown in the Examples, a total cellular extract can be prepared for cells of the sample, resolved by a SDS-PAGE electrophoresis and submitted to immunoblot analysis with NFAT antibodies for changes in mobility shifts directly associated with phosphorylation levels.

In order to determine whether calcineurin has dephosphorylated NFAT, a radioactively labelled phosphate group may also be used, e. g. in the form of ³²P-orthophosphate. This will provide a direct signal on NFAT which may be determined by counting incorporated radiolabel or other means, such as immuno- precipitating NFAT, separating NFAT on a gel and subjecting the gel to autoradiography to determine the signal from NFAT.

In an other aspect, the methods can use a conformational antibody which distinguishes between phosphorylated NFAT and un-phosphorylated NFAT. Such antibodies, which may be polyclonal, monoclonal or binding fragments of complete antibody molecules (e. g. single chain Fv fragments) may also be used in determining the extent to which the residue has been phosphorylated. Kits comprising such antibodies form another aspect of the invention.

### EXAMPLES

The following example illustrates the invention.

### MATERIALS AND METHODS

### Mouse models:

The following mouse models of human lymphoma/leukemia were used in the present study. The generation of transgenic mice that constitutively express TEL-JAK2 under control of the lymphoid lineage specific EµSRα promoter have been previously described by the inventors' group (18). TEL-JAK2 transgenic mice develop a fatal T-cell acute lymphoblastic leukemia (T-ALL) at 2 to 22 weeks of age with specific amplification of the SP CD8 and DP CD4/CD8 lymphoid T-cells. Leukemogenic potential of Notch1 was formaly proven in studies published by Pear and colleagues using a bone marrow reconstitution assay with cells containing a retrovirally-transduced, activated-form of Notch1, ICN1 (15). 100% of the animals reconstituted with cells expressing ICN1 allele developed DP CD4/CD8 T-ALL by 3 to 8 weeks post transplantation. Retroviral-mediated transfer of ICN1 in mouse bone marrow HSCs followed by adoptive transfer in irradiated recipient mice was as described. T cell leukemia developped in spleen and lymph nodes of recipient mice within 1 month as originally described. The transgenic line in which a tamoxifen inducible Myc fusion protein (c-Myc-ER) is expressed in the T cell lineage under control of the CD2 promoter has been previously described. In the present study, the inventors have used a cell line derived from a c-Myc-ER-induced thymic lymphoma in p53+/- mice, ERP15-14 (kindly provided by Dr. J. Neil). These cells were maintained either in tissue culture, or transplanted in nu/nu mice where they formed tumors at the site of injection. The tumor cells obtained from a human EBV-induced B cell lymphoma have been propagated in SCID mice and were kindly provided by Dr. D. Decaudin (Institut Curie, Paris).

### Cell culture

Leukemia-derived primary cells and leukemia cell lines (TEL-JAK2, ICN1 and ERP15-14) were maintained in RPMI 1640 supplemented with 10% foetal calf serum, 100U/ml penicillin, 100µg/ml streptomycin, 2mM glutamine (all from Life Technologies) and 5.10⁻⁵ 2-βmercaptoethanol (Sigma). The ERP15-14 cell line was maintained in the absence of 4-hydroxy-tamoxifen (40HT, from Sigma), suggesting that the Myc-ER transgene shows basal expression activity in the absence of exogenous stimulation with 4-OHT. Cyclosporin A (CsA) and ionomycin (Iono) used for in vitro experiments have been purchased from Sigma and used at 1 µg/ml for the period of time indicated (CsA cat. C1832; Iono cat. I0634).

### In vivo drugs administration

Cyclosporin A (Neoral100mg/ml, Novartis) or FK506 (PROGRAF, for intravenous injection 5mg/ml, Fujisawa Laboratories) have been diluted in 10% Cremophor (BASF). C57BL6 mice at 8 weeks of age were injected via the caudal vein with 5.10⁶ TEL-JAK2 leukemic cells. One week after injection, one mouse was sacrified to ascertain that leukemic cells had invaded the spleen of recepients mice. At that time 3 groups of mice were randomly selected and implanted with osmotic pumps (ALZET) containing either CsA (0.6mg/mouse/day), Prograf (0.06 mg/mouse/day) or left untreated. The osmotic pump system have been used to insure continous delivery of the drugs and to avoid the toxic effects observed with acute delivery via daily i.p injections. Finally, 5 or 10 days post-treatment, mice were sacrified and subjected to analysis.

### Analysis of NFAT activation in leukemic cells by EMSA.

A [³²P]dCTP end-labeled probe corresponding to the mouse IL2 -45 promoter region (+ strand : 5'-cgagaatgctGGAAAaataatatgggggtg-3' (SEQ ID No 1) was used to evaluate NFAT DNA-binding activity by Electrophoresis Mobility Shift Assay (EMSA), as described previously, using 2 µg proteins from nuclear extracts prepared from TEL-JAK2 leukemic T cells obtained from invaded thymuses and, as control, thymocytes from non transgenic littermates.

### Analysis of calcineurin/NFAT activation by western blot.

Total cellular extracts prepared from cells obtained directly from diseased animals or control littermates or harvested at the indicated times following culture, were resolved by SDS-PAGE and subjected to immunoblot analysis with the indicated antibody (Ab). The NFAT1(sc-7296), NFAT2 (sc-7294), NFAT4 (sc-8321), STAT5 (C-17; sc-835) antibodies were purchased from Santa Cruz Biotechnology. The phosphotyrosine- STAT5 antibody (05-495) was purchased from Upstate Biotechnology. The pan-NFAT Ab (796) was kindly provided by Dr. Nancy Rice.

### RESULTS and DISCUSSION.

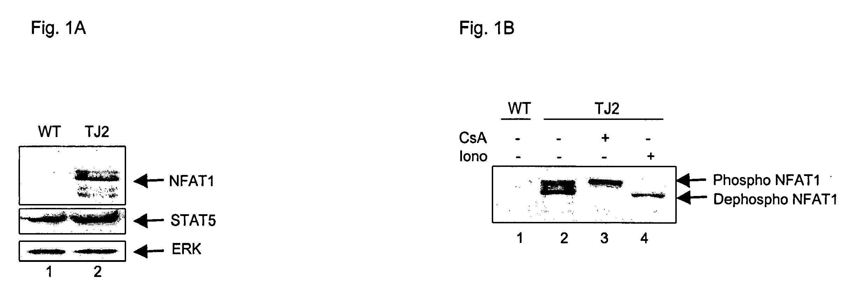

The status of NFAT protein expression and calcineurin activation in TEL-JAK2 leukemic cells was analyzed by western blot, using antibodies specific for NFAT1, NFAT2 and NFAT4 and compared to control thymocytes. The state of NFAT activation can be easily assessed by SDS/PAGE since the fully dephosphorylated form (activated form) of the respective NFATs migrate faster in these conditions than the phosphorylated NFAT isoforms, the fully phosphorylated form displaying the slowliest migration. The results of Figure 1 show that TEL-JAK2 leukemic cells obtained from an invaded thymus of a diseased TgTEL-JAK2 mouse express higher levels of NFAT1 as compared to normal thymocytes obtained from a non transgenic littermate control (Fig.1A). Furthermore, NFAT1 was essentially stoechiometrically present in its fully dephosphorylated (activated) state in leukemic cells as shown by its rapid electrophoretic migration. For comparison, Fig. 2B displays the relative migration of the fully dephosphorylated and fully phosphorylated NFAT1 isoforms, obtained from TEL-JAK2 leukemic cells maintained in culture for 1 hours in the presence of CsA to inhibit calcineurin to basal levels of activity, or in the presence of ionomycin to optimally activate calcineurin. As can be observed from this analysis, the NFAT1 isoform observed in TEL-JAK2 leukemic cells migrates at the same position as the fully activated NFAT induced in ionomycin-treated cells (Fig 1B, compare lanes 2 to 4). The use of antibodies specific for NFAT2 and NFAT4 similarly demonstrated the activation of these NFAT proteins in TEL-JAK2 leukemic cells as compared to normal thymocytes control (data not shown). However, unlike NFAT1, the expression levels of NFAT2 and NFAT4 were found to be similar in TEL-JAK2 leukemic cells as compared to control thymocytes (data not shown). To independently demonstrate the activation of the calcineurin/NFAT pathway in TEL-JAK2 leukemic cells, the inventors compared the NFAT DNA binding activity by electrophoretic mobility shift assay (EMSA) in nuclear extracts obtained from TEL-JAK2 leukemic cells and normal thymocyte, as control. The probe used in these experiments was a high affinity [³²P]-labelled DNA oligonucleotide corresponding to the - 45 NFAT binding site of the mouse IL2 promoter. As shown in Fig.2A, almost no retarded complex could be detected in thymocyte nuclear extracts, reflecting the low, steady-state levels of NFAT activation in developping thymocytes. In contrast, TEL-JAK2 nuclear extracts displayed a high level of DNA binding activity to the NFAT probe (Fig.2A, compare lanes 2 and 3). This difference did not result from a difference in nuclear protein concentration between leukemic and control cells, since the same level of DNA binding activity to an Sp1-specific probe was observed in both types of extracts (Fig.2A. bottom panel). The NFAT/probe complex was specific as its formation was inhibited by the addition to the reaction mixture of a 100 fold molar excess of unlabeled NFAT oligonucleotide used as competitor, but was unaffected in the presence of the same molar excess of a mutant NFAT oligonucleotide carrying a mutation in the NFAT binding site core sequence (data not shown). The NFAT/probe complex was quantitatively super-shifted by the addition to the reaction mixture of an antibody specific to an epitope common to NFAT1-4 (pan-NFAT antibody), but not by a control antibody (Fig.2B, compare lanes 2, 5 and 6). In line with the fact that NFAT1 is overexpressed in TEL-JAK2 leukemic cells, most -but not all- of the NFAT/probe complex was supershifted by addition of an excess of NFAT1-specific antibody (Fig.2B, compare lanes 2 and 3). In contrast, the antibody directed against NFAT4 only slightly affect the complex, suggesting that NFAT4 is contributing to NFAT DNA binding activity observed in TJ2 leukemic cells but to a lesser extend as compared to NFAT1. Similar observations were made in several pairwise comparison between control thymocytes and independent TEL-JAK2 leukemias arising in different TgTEL-JAK2 mouse individuals (data not shown). The inventors conclude from these experiments that TEL-JAK2 leukemic cells both upregulate the expression of NFAT1 and display the constitutive dephosphorylation, nuclear accumulation and DNA binding activation of NFAT1, NFAT2 and NFAT4.

In order to investigate whether constitutive NFAT activation was specific to TEL-JAK2 leukemia or whether it is a more general property of leukemic cells, the inventors analyzed NFAT protein expression and activation in other mouse models of human leukemia. Mutation of Notch1 by either point mutation or as the result of the t(7;9)(q34;q34.3) chromosomal translocation is observed in a majority of human T cell leukemia. Previous studies have shown that retroviral-mediated transduction of mouse bone marrow cells with an activated Notch mutant (Intracellular Notch1= ICN1) followed by adoptive transfer of transduced cells in irradiated syngeneic hosts resulted in a T cell lymphoma/leukemia that faithfully reproduced the human disease (15). ICN1-induced leukemia were generated using this protocol and analyzed for NFAT activation as described above. As shown in Fig. 3A, ICN1-induced leukemic cells expressed the dephosphorylated (activated) isoforms of NFAT1 (Fig3A, upper panel, compare lanes 1 to 7), NFAT2 (Fig.3A, bottom panel, compare lanes 1 to 7) and NFAT4 (data not shown). Cyclosporin A treatment of ICN1 leukemic cells lead to the appearence of hyperphosphorylated (inactived) isoforms of NFAT2 at the expense of the non phosphorylated isoforms that are not observed in non-treated ICNI leukemic cells (Fig.3A bottom panel, compare lanes 1-7 to lane 8 ; see Fig. 3B for a scheme). In contrast, ionomycin-treated ICN1 leukemic cells show an NFAT2 migration profile which is indistinguishable from that observed in ICN1 non-treated leukemic cells (Fig. 3A, bottom panel, compare lanes 1-7 to lane 9; see Fig. 3B for a scheme). These results show that NFAT proteins are in their fully activated state in ICN1 leukemic cells. Similar observations were made in transplanted T cell leukemia obtained following inoculation of CD2-Myc-induced T cell leukemia to nu/nu recipient mice as well as in a mouse xenograft model of a human Hodgkin-like B cell lymphoma (Fig.5B).

The fact that NFAT activation is observed in a large panel of lymphoid malignancies, induced by primary oncogenes acting in distinct signaling networks suggested to us that it was unlikeky to result from the activity of the initiating oncogene. To investigate this in further detail, the inventors compared NFAT1 activation in extracts of leukemic cells obtained directly from diseased animals, or from the same cells maintained in culture in the absence of growth factors and serum (Fig. 4A and B, compare lanes 2 to 3). Under these consitions, TEL-JAK2 tyrosine kinase activity is not affected as shown by the maintenance of STAT5 in its tyrosine-phosphorylated state (Fig. 4B). In line with the results described above, NFAT1 was in its dephosphorylated (activated) state in TEL-JAK2 leukemic cells obtained directly from diseased animals. In contrast, maintenance of these cells in culture resulted in their stoechiometric rephosphorylation (inactivation) by the endogenous, NFAT protein kinases (Fig. 4A compare lanes 2 and 3). As expected, re-phosphorylation of NFAT1 lead to a decrease in DNA binding activity in leukemic cells to the levels normally observed in normal thymocytes, as analyzed by EMSA (data not shown). Similar to the results described above for TEL-JAK2 leukemic cells (Fig. 4A), ICN1-induced leukemias (Fig. 5A) and human EBV-induced B cell lymphoma (Fig. 5B) displayed the activated isoform of NFAT1 when leukemic cells were obtained directly from diseased animals and NFAT activation was lost when cells were maintained in culture for a period of time as short as one hour. These results indicate that NFAT activation is not under the sole control of the initiating oncogene of these leukemia and appears to require the presence of a proper in vivo tumor microenvironment.

To investigate whether activation of the calcineurin/NFAT pathway is important for tumor maintenance in vivo and to test whether the well characterized calcineurin inhibitors currently used in human medicine could be of therapeutic value, the inventors analyzed the in vivo effects of both CsA and FK506 on TEL-JAK2 leukemia progression. Primary TEL-JAK2 leukemic cells were grafted by i.v. inoculation to syngeneic recipient mice. Under these conditions, leukemia corresponding to the expansion of the original leukemic clone efficiently transplanted in secondary hosts to invade their spleen and lymph nodes and to induce their death within 20-30 days. Recipient mice were transplanted with TgTEL-JAK2 leukemic cells and maintained for one week to allow moderate leukemic cell expansion. After that period of time, three cohorts were generated. The first was left untreated, the second group was implanted with an osmotic pump delivering a continuous amount of CsA and the third implanted with osmotic pumps delivering FK506 (see Materials and methods).

When compared to non-treated control mice, 10 days CsA and Prograf-treated mice exhibited a statistically reduced spleen weight (Fig. 6A and 6B; p value<0.05), suggesting that CsA treatment either induced apoptosis of leukemic cells and/or inhibited their proliferation. To analyze this in further details, bone marrow imprints from untreated or CsA- or Prograf-treated leukemia were morphologically analyzed. Histopathological analysis of the liver parenchyme of these mice was also carried out. Figure 8 show that bone marrow from leukemic TgTEL-JAK2 mice was exclusively composed of an homogenous population of T lymphoblastic cells (Fig. 8B) while normal bone marrow is mainly composed of granulocytic cells. Interestingly, treatment of mice with either CsA or Prograf resulted in the severe decrease in the number of leukemic blasts and in the recovery of a cell composition close from that of normal bone marrow (Fig. 8C and 8D). The process of tumor metastasis was also strongly inhibited by CsA or Prograf treatment. Indeed, whereas leukemic blasts efficiently invaded the liver sinusoids and parenchyma of non-treated mice (Fig. 9, panels A and B), leukemic blasts were severely reduced in numbers in the livers from CsA- or Prograf-treated mice (Fig. 9C and 9D).

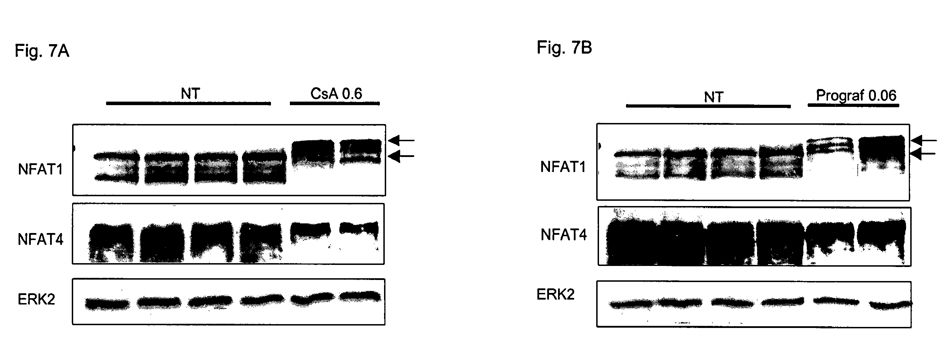

Biochemical analysis of leukemic cells isolated from the spleen of non-treated mice and from mice treated with either CsA or Prograf showed the loss of NFATs activation as shown by the appearence of slowly migrating, heavily phosphorylated (inactive) isoforms of NFAT1 and NFAT4 (Fig. 7A and 7B), demonstrating that NFAT activation in TEL-JAK2 leukemia is under control of calcineurin and that inhibition of this process by CsA or Prograf (FK506) is associated with the inhibition of tumor growth progression. Of note, constitutive activation of NFκB, another REL superfamily member activated in these leukemic cells (N. dos Santos and JG, unpublished obs.) was not affected by treatment with CsA or Prograf, demonstrating the specificity of these compounds for the calcineurin/NFAT pathway (data not shown).

In conclusion, these results show (i) that the calcineurin/NFAT pathway is activated in a variety of mouse models for T and B cell lymphoma/leukemia, a property which begins to be recognized in human lymphoid malignancies as well (inventors' unpublished observations; (13) ; (ii) that activation of this pathway is observed in lymphoid malignancies initiated by a wide spectrum of iniating oncogenes and depends upon the presence of a specific in vivo environment; (iii) that activation of the calcineurin/NFAT pathway is important for leukemia progression in vivo ; (iv) that pharmacological inhibitors of calcineurin activity, namely cyclosporin A and Prograf (FK506) are of therapeutical benefit in mouse models of human leukemia. The inventors propose that targeting the calcineurin/NFAT signaling pathway for inhibition by treatment by CsA or/and FK506 (Prograf), two calcineurin inhibitors commonly used in transplantation medicine can be of therapeutical benefit in curative treatment of human lymphoid malignancies by affecting the leukemic cell itself and/or its microenvironment (stroma ; angiogenesis). Since activation of this pathway is not necessarily under control of the primary (initiating) oncogenic, activation of the calcineurin/NFAT pathway may be a valuable marker of tumor evolution (stage) and/or lymphoma/leukemia classification significant to prognosis and/or diagnosis. Finally the animal models and approaches used here paves the way to identify and study novel inhibitory compounds of calcineurin and/or NFATs in hematopoietic malignancies.

### REFERENCES

All documents mentioned in the specification are incorporated herein by reference.
1. Klee, C. B., Ren, H., and Wang, X. (1998) J Biol Chem 273(22), 13367-13370
2. Graef, I. A., Chen, F., Chen, L., Kuo, A., and Crabtree, G. R. (2001) Cell 105(7), 863-875
3. Hogan, P. G., Chen, L., Nardone, J., and Rao, A. (2003) Genes Dev 17(18), 2205-2232
4. Okamura, H., Aramburu, J., Garcia-Rodriguez, C., Viola, J. P., Raghavan, A., Tahiliani, M., Zhang, X., Qin, J., Hogan, P. G., and Rao, A. (2000) Mol Cell 6(3), 539-550
5. Peng, S. L., Gerth, A. J., Ranger, A. M., and Glimcher, L. H. (2001) Immunity 14(1), 13-20
6. Oukka, M., Ho, I. C., de la Brousse, F. C., Hoey, T., Grusby, M. J., and Glimcher, L. H. (1998) Immunity 9(3), 295-304
7. Macian, F. (2005) Nat Rev Immunol 5(6), 472-484
8. Masri, M. A. (2003) Mol Immunol 39(17-18), 1073-1077
9. Aramburu, J., Yaffe, M. B., Lopez-Rodriguez, C., Cantley, L. C., Hogan, P. G., and Rao, A. (1999) Science 285(5436), 2129-2133
10. Roehrl, M. H., Kang, S., Aramburu, J., Wagner, G., Rao, A., and Hogan, P. G. (2004) Proc Natl Acad Sci U S A 101(20), 7554-7559
11. Neal, J. W., and Clipstone, N. A. (2003) J Biol Chem 278(19), 17246-17254
12. Jauliac, S., Lopez-Rodriguez, C., Shaw, L. M., Brown, L. F., Rao, A., and Toker, A. (2002) Nat Cell Biol 4(7), 540-544
13. Marafioti, T., Pozzobon, M., Hansmann, M. L., Ventura, R., Pileri, S. A., Roberton, H., Gesk, S., Gaulard, P., Barth, T. F., Du, M. Q., Leoncini, L., Moller, P., Natkunam, Y., Siebert, R., and Mason, D. Y. (2005) Br J Haematol 128(3), 333-342
14. Carron, C., Cormier, F., Janin, A., Lacronique, V., Giovannini, M., Daniel, M. T., Bernard, O., and Ghysdael, J. (2000) Blood 95(12), 3891-3899
15. Pear, W. S., Aster, J. C., Scott, M. L., Hasserjian, R. P., Soffer, B., Sklar, J., and Baltimore, D. (1996) J Exp Med 183(5), 2283-2291
16. Kurreck, J. (2003) Eur J Biochem. 270(8), 1628-44
17. Usman, N., Blatt, L.M. (2000) J Clin Invest. 106(10), 1197-202
18. Mittal, V. (2004) Nat Rev Genet 5(5): 355-65

## Claims

1. Use of a drug inhibiting the calcineurin and/or the calcineurin/NFAT pathway for the preparation of a medicament for treating a haematopoietic tumor.

2. Use according to claim 1, wherein said drug inhibiting the calcineurin and/or the calcineurin/NFAT pathway is used in combination with a cancer therapy.

3. Use according to claim 2, wherein said cancer therapy is selected from the group consisting of a cancer chemotherapy, an immunotherapy, a radiotherapy, a hormone or cytokine therapy, any other therapeutic method used for the treatment of a haematopoietic tumor and a combination thereof, preferably a cancer chemotherapy.

4. Use of a drug inhibiting the calcineurin and/or the calcineurin/NFAT pathway for the preparation of a medicament for increasing the efficiency of a treatment of a haematopoietic tumor.

5. Use according to any one of claims 1 to 4, wherein said drug inhibiting the calcineurin and/or the calcineurin/NFAT pathway is a drug that inhibits NFAT dephosphorylation, such as drug that inhibits calcineurin, a drug that inhibits the interaction between calcineurin and NFAT, and a drug that activates a NFAT kinase.

6. Use according to claim 5, wherein said drug inhibiting the calcineurin is a calcineurin inhibitor.

7. Use according to claim 6, wherein said calcineurin inhibitor is cyclosporin A or FK506.

8. Use according any one of claims 1 to 7, wherein said hematopoietic tumor is a lymphoma and/or a leukemia.

9. Use according any one of claims 1 to 8, wherein the subject to be treated presents dephosphorylated NFAT in cells of the haematopoietic tumor isolated from said subject.

10. A pharmaceutical composition comprising a drug inhibiting the calcineurin and/or the calcineurin/NFAT pathway and an anticancer drug.

11. Product containing a drug inhibiting the calcineurin and/or the calcineurin/NFAT pathway and an anticancer drug as a combined preparation for simultaneous, separate or sequential use in the treatment of a hematopoietic tumor.

12. Pharmaceutical composition according claim 10 or product according to claim 11, wherein said drug inhibiting the calcineurin and/or the calcineurin/NFAT pathway is a drug that inhibits NFAT dephosphorylation, such as drug that inhibits calcineurin, a drug that inhibits the interaction between calcineurin and NFAT, and a drug that activates a NFAT kinase.

13. Pharmaceutical composition or product according to claim 12, wherein said drug inhibiting the calcineurin and/or the calcineurin/NFAT pathway is a calcineurin inhibitor, such as cyclosporin A or FK506.

14. A method for staging or characterizing a hematopoietic tumor in a subject comprising determining the phosphorylation state of NFAT in a cancer sample isolated from said subject.
